# EUROPEAN PATENT APPLICATION

(11) **EP 1 213 299 A1**
(43) Date of publication of application: **12.06.2002**
(21) Application number: 00204398.2
(22) Date of filing: 08.12.2000
(51) Int. Cl.: C07K 14/025, A61K 38/17, A61K 39/12

(54) **Immunogenic epitopes of human papilloma virus and uses thereof**

(71) Applicant: Leids Universitair Medisch Centrum, 2333 ZA Leiden (NL)
(72) Inventor: The designation of the inventor has not yet been filed
(74) Representative: Prins, Adrianus Willem

(57) **Abstract**

The invention is concerned with epitopes derived from human papilloma virus and peptides comprising said epitopes. Epitope specific T cells are generated, induced and/or triggered from memory. The invention further provides clinically relevant approaches for immunizing, vaccinating subject against HPV. The invention also provides methods and uses suited to treat subjects suffering from grade III (CIN III) lesions and/or cervical cancer.

## Description

**Technical Field:** This invention relates generally to the field of medicine, and more specifically to specific epitopes useful in the treatment and diagnosis of cervical cancers which express the human papilloma virus oncoprotein.

**Background:** The majority of cervical cancers express the HPV16-derived E6 and E7 proteins (Bosch *et al.,* 1995). Therefore, these proteins are excellent target antigens for tumor-specific immunity. Considerable interest exists in the identification of epitopes involved in the immune response to HPV16, given the possibility to incorporate these as subunits into a vaccine or to use these epitopes to monitor vaccine induced immunity *in vivo*. Since most epithelial cells express MHC class I but not class II, the attention has so far been focused on the induction of tumoricidal HPV-specific CD8+ cytotoxic T lymphocytes (Melief *et al.,* 2000; Ressing *et al., 1995;* Ressing *et al.,* 2000; Ressing *et al.,* 1996). HPV-specific CD8+ T-cell reactivity has been found in the peripheral blood of patients diagnosed with cervical intraepithelial neoplasia grade III (CIN III) lesions or cervical carcinoma (Nimako *et al.,* 1997; Ressing *et al.,* 1996) and in tumor-infiltrating T-cell populations isolated from patients with cervical cancer (Evans *et al.,* 1997).

Tumor-specific CD4+ T helper ("Th") immunity is now also considered pivotal for the efficient eradication of solid tumors, despite the fact that most of these tumors do not express MHC class II (reviewed in Melief *et al.,* 2000; Pardoll and Topalian, 1998; Toes *et al.,* 1999). A positive role for HPV-specific Th-immunity was suggested by the predomination of CD4+ T-cells in regressing genital warts (Coleman *et al.,* 1994) as well as by the detection of delayed-type hypersensitivity responses to HPV16 E7 in the majority of subjects with spontaneous regressing CIN lesions (Hopfl *et al.,* 2000). Furthermore, HPV16-specific CD4+ T-cells have been detected in the blood of patients with persistent HPV infections, high-grade CIN lesions or cervical cancer (de Gruijl *et al.,* 1998). For the less prevalent oncogenic types HPV59 and HPV68, HLA-DR4 restricted Th-cells were isolated from the T-cells that infiltrated a cervical cancer lesion (Hohn *et al.,* 1999). In contrast, despite the presence of HPV16 in the majority of cervical cancers, no in depth information is available concerning HLA-restriction and epitope specificity of HPV16-specific T-helper responses.

### Disclosure of the Invention

We hereby present the first detailed analysis of Th-immunity against E7 of the prevalent high risk type HPV16, restricted by common MHC class II molecules. We identify the most immunogenic regions within the E7 sequence, as well as a number of naturally processed Th-epitopes mapping in this region, using respectively short and long-term PBMC cultures derived from healthy blood donors. In parallel, the *in vivo* induced E7-specific immunity, as detected by IFNγ ELISPOT assays, was analyzed in subjects diagnosed with HPV16+ lesions.

Tumor-specific T helper immunity was found to play a pivotal role in the natural and vaccine-induced immune defense against tumors. We investigated the Th response against the human papilloma virus type 16 (HPV16) E7 oncoprotein in detail. By means of PBMC cultures from HLA-typed healthy donors, we identified the central part of HPV16 E7 (E7₄₁₋₇₂) as the major immunogenic region within this antigen. Furthermore, we mapped three distinct Th-epitopes within this region (DR15/E7₅₀₋₆₂, DR3/E7₄₃₋₇₇, DQ2/E7₃₅₋₅₀).

In a parallel approach, employing IFNγ ELISPOT analysis, we detected Th-immunity against HPV16 E7 in subjects with HPV16+ lesions. Several of these responses matched with the three Th-epitopes defined in this study. A number of other HPV16+ subjects did not display any E7-specific type 1_cytokine producing T cell immunity, indicating failure of the immune response.

### Brief Description of the Figures

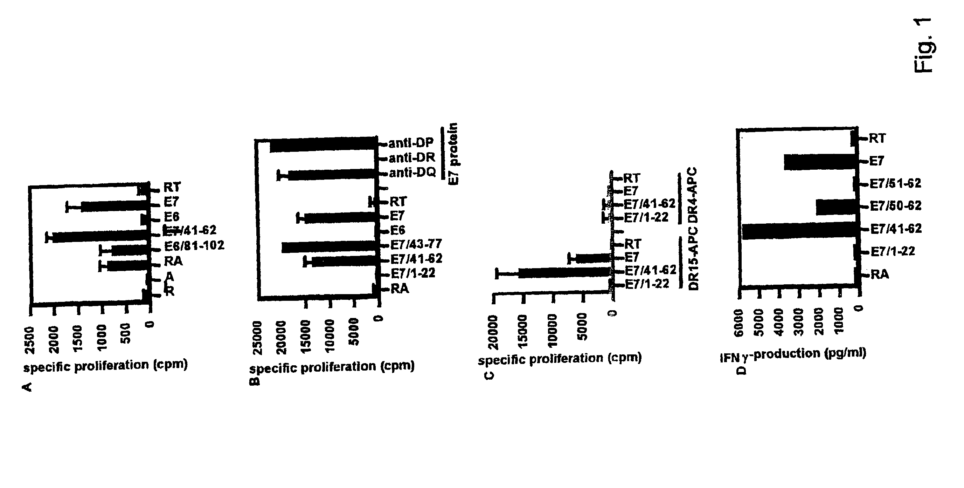

FIG. 1 depicts the peptide E7₄₁₋₆₂ induced T-cell response that recognizes naturally processed Ag. PBMC from a healthy HLA-DR15, 4 and DQ6,7 positive blood donor were stimulated four times with peptide E7₄₁₋₆₂. The responding T-cells were tested in a 3-day proliferation assay (a, b, c) or stimulated for 1 day in order to measure the production of IFNγ by ELISA (d). Responder cells (R) and autologous or MHC class II matched antigen presenting cells (A) were incubated with the indicated Ags: recombinant HPV16 E6 protein (E6), recombinant HPV16 E7 protein (E7), recombinant HIV RT protein (RT) or peptides derived from E6 (eg E6/81-102, peptide E6₈₁₋₁₀₂) or derived from E7. Proliferation of the bulk T-cell culture (a), proliferation of the T-helper clone, derived from the bulk T-cell culture, and blocking of the E7 protein-specific response by adding an antibody against HLA-DR (b), proliferation of the T-helper clone when stimulated with partially MHC class II matched antigen presenting cells (c), and fine mapping of the minimal epitope by measurement of the IFNγ production per 24 h by the T-helper clone (d).

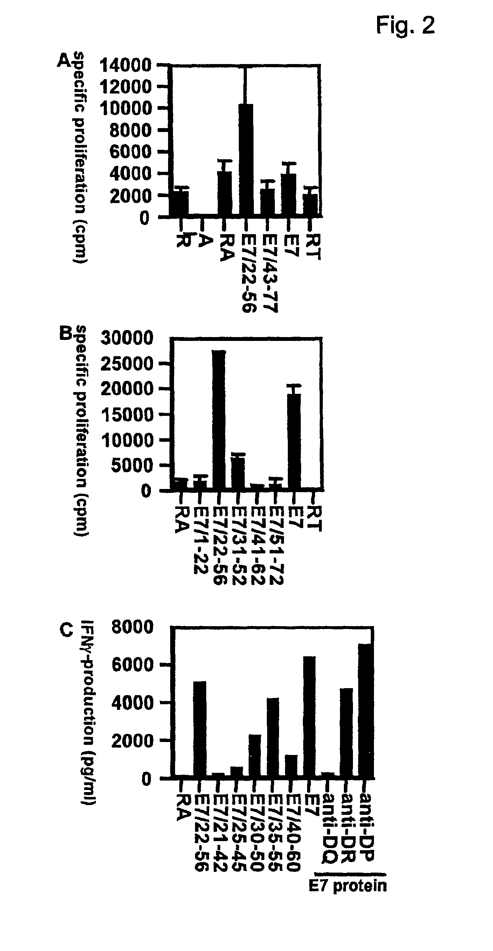

FIG. 2 depicts a peptide E7₂₂₋₅₆ induced T-cell response that recognizes naturally processed Ag. PBMC from a healthy HLA-DR3 and DQ2 positive blood donor were stimulated four times with peptide E7₂₂₋₅₆. Proliferation of the bulk T-cell culture (a), specific proliferation of the T-helper clone, derived from the bulk T-cell culture, when stimulated with E7₂₂₋₅₆, E7₃₁₋₅₂ and E7 protein (b), and fine mapping of the minimal epitope and MHC class II restriction by measurement of the IFNγ production per 24 h of the T-helper cell clone (c). *(See, also,* FIG. 1 and associated description).

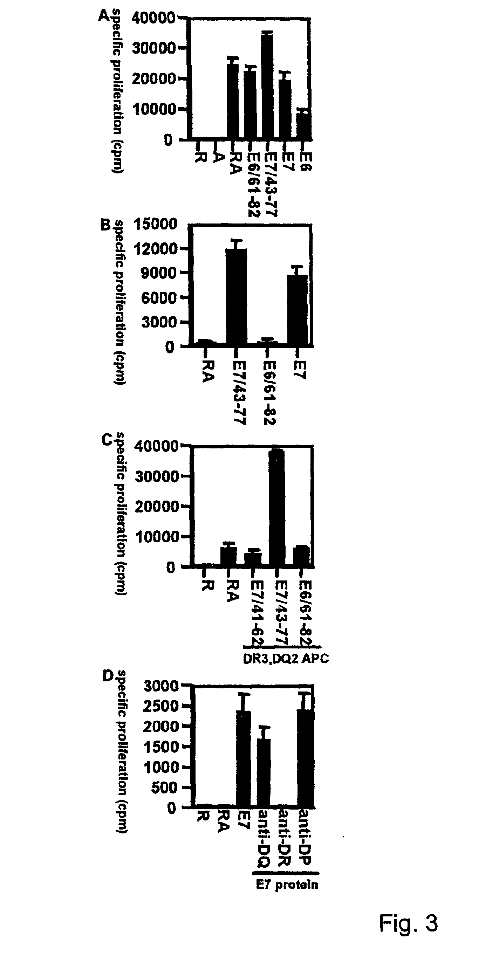

FIG. 3 depicts a peptide E7₄₃₋₇₇ induced T-cell response that recognizes naturally processed Ag. PBMC from a healthy HLA-DR1,3 and DQ2 positive blood donor were stimulated four times with peptide E7₄₃₋₇₇. Proliferation of the bulk T-cell culture (a), specific proliferation of the T-helper cell clone against peptide E7₄₃₋₇₇ and E7 protein (b), recognition of partially matched (DR3,DQ2) peptide pulsed antigen presenting cell ("APC") (c) anti HLA-DR antibody blocks the E7-specific proliferation of the T-helper cell clone revealing HLA-DR3 as restriction element (d), and specific production of IFNγ by the T-helper cell clone when stimulated with E7₄₃₋₇₇ peptide or E7 protein (e). *(See, also,* FIG. 1 and associated description).

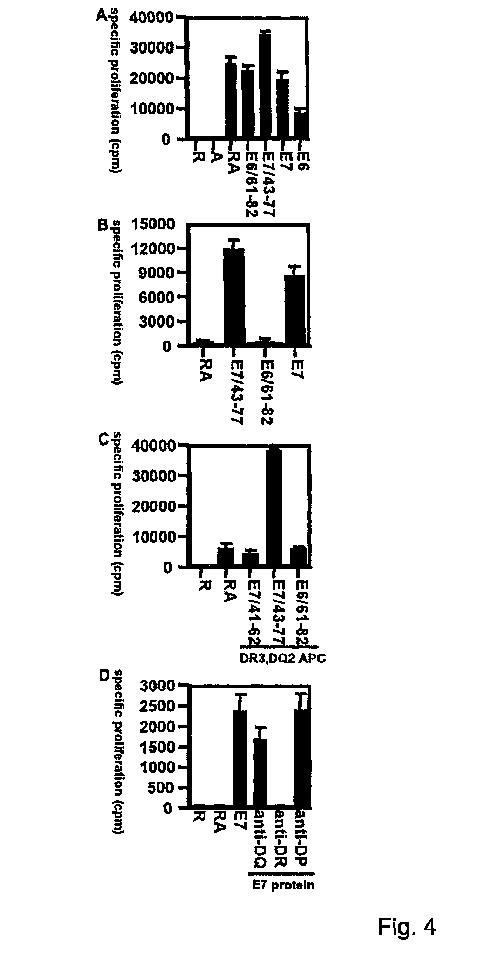

FIG. 4 depicts the stimulation with influenza matrix 1 (M1)-peptides of MACS-separated CD45RA+ (naive) T-cells *(left)* and CD45RO+ (memory) T-cells *(right)* results in the production of IFNγ in the CD45RO+ subset only. The M1 protein was divided in 16 overlapping peptides. Each pool consists of four 30 amino acid long peptides that overlap by 15 amino acids. Tet. tox.: tetanus toxoid.

### Detailed Description of the Invention

The invention provides an immunogenic epitope selected from the central part of a human papilloma virus 16 E7 protein. Preferably, said central part comprises a region spanning amino acid 35-77 of a human papilloma virus 16 E7 protein. More preferably said central part comprises a region spanning amino acid 35-50, a region spanning amino acid 50-62 and/or a region spanning amino acid 43-77 in said E7 protein. Said epitope may be presented in any form. Preferably, said epitope is presented in the form of a peptide. Thus in one embodiment the invention provides a peptide comprising an immunogenic epitope of the invention. The art currently knows many way of generating a peptide. The invention is not limited to any form of generated peptide as long as the generated peptide comprises an epitope of the invention. By way of example, a peptide of the invention can be obtained from protein E7, synthesized in vitro or by a cell for instance through an encoding nucleic acid. A peptide of the invention can be present as a single peptide or incorporated into a fusion protein. In one embodiment said peptide is flanked by processing sites allowing processing of said peptide inside a cell such as to allow transport and/or incorporation into an MHC molecule on the surface of said cell. In a preferred embodiment a peptide of the invention is capable of complexing with an MHC class II molecule. MHC class II is currently considered to be important in eradication of for instance tumor cells although said tumor cells often do not express MHC class II.T cell mediated immunity. Peptides of the invention a particularly well suited for eliciting, inducing and/or stimulating MHC class II dependent T cells. At least some peptides of the invention appear to connect to memory T cells of subjects infected with HPV-16. Reactive T cells have been found in subjects comprising CIN III lesions and in subjects having cervical cancer. This indicates that the identified central part of the HPV16 E7 protein comprises epitopes relevant for anticancer treatments based on vaccination and/or adoptive immunotherapy approaches.

Epitopes of the invention are suited for the generation and/or induction of HPV specific T-cells. In one aspect the invention therefore provides a method for generating a human papilloma virus 16 specific T-cell comprising contacting a collection of naïve T-cells with a peptide of the invention and culturing at least part of said collection of T-cells. In another aspect the invention provides a method for inducing IFNγ production by a human papilloma virus 16 specific memory T-cell comprising contacting a collection of T-cells of a subject infected with human papilloma virus 16 with a peptide of the invention and culturing at least part of said collection of T-cells. In a preferred embodiment of these aspects said human papilloma virus 16 specific T-cell is isolated.

In yet another aspect the invention provides an isolated T cell obtainable by a method of the invention. Said T cell can be used in many way, for instance for the detection of an epitope of the invention for instance in a diagnostic assay. However, preferably said T cell is used in immunotherapy approaches. Such approaches incorporate but are not limited to approaches wherein such T cells are infused into a subject suffering from an HPV-16 induced cervical cancer or lesion. In such approaches it is preferred that said T cell and said subject are histocompatible. Although this may not always be true. In some cases mismatches for histocompatible even enhance the functionality of said T cell in said subject. The risk of developing graft versus host disease can often be limited by assuring that T cells not restricted to an epitope of the invention are not present in a graft given to said subject. Of course, host versus graft responses resulting in elimination of infused T cells of the invention can occur. To limit such responses histocompatibility is preferred. However, in case wherein irradiation of tumor cells is very rapid, host mediated removal of grafted cells can be an advantage as an additional safety feature of immunotherapy. In one embodiment the invention therefore provides a method for eliciting and/or enhancing an HPV16 E7 protein specific immune response in an individual comprising administering to said individual a peptide of the invention or a T-cell of the invention. A peptide of the invention is particularly suited to elicit HPV 16 specific immune responses in an individual. These immune responses can be sufficiently high to provide a subject with protection against infection by HPV-16. However, a peptide or a T cell of the invention can also be used to aid in combating an already present infection in a subject. Thus in another embodiment the invention provides an a vaccine comprising a peptide of the invention and a suitable carrier. In another embodiment the invention provides the use of a T cell of the invention for immunotherapy. In a one embodiment said vaccine is used for the treatment of a subject comprising an HPV16 lesion.

In another aspect the invention provides a method for determining whether a collection of T cells comprises a memory T cell specific for an epitope comprising providing said collection of T cells with a peptide comprising said epitope, culturing said collection of T cells in the presence of an IFNγ antibody and detecting any bound IFNγ. In a preferred embodiment said IFNγ antibody is bound to a solid phase, preferably a solid surface.

The invention is further explained by the use of the following illustrative example.

### Example

### Material and Methods

### Subjects and Controls.

Samples of umbilical cord blood mononuclear cells (CBC) were used as immunologically naive controls for influenza matrix-specific responses. PBMC of HLA-typed, anonymous healthy blood bank donors (D) obtained after informed consent, served as control PBMC for HPV16 E7 and influenza matrix-specific responses. Since these donors are anonymous, no additional data is available. However, donors with a known recent history of infection, including abnormal pap-smear, were, as part of normal regulations, discouraged to donate blood. The study of subjects (S; Table II) with CIN or cervical carcinoma in this paper was nested in the "CIRCLE study" that investigates cellular immunity against HPV16 infected cervical lesions. Women presenting with histologically proven CIN III or cervical carcinoma at the department of gynecology of the Leiden University Medical Center (LUMC) were, after informed consent, enrolled in this study. The study design was approved by the ethics committee of the LUMC. Blood was drawn at day of treatment. Subjects with CIN III were treated by LEEP or cold knife conization. In case of stage IB-IIA a radical hysterectomy was performed. All individuals for whom enough PBMC were available were typed for HLA MHC class II (Naipal *et al.,* 1984). Subjects were typed for HPV16 using HPV16-specific primers on DNA isolated from paraffin-embedded sections of biopsies or surgical resection specimens (Claas *et al.,* 1989). Since HPV-specific T-helper response were expected to be found in subjects with progressive disease (de Gruijl *et al.,* 1998), we chose to analyze three subjects presenting with CIN III, 4 subjects with stage IB cervical cancer and 4 subjects with recurrent cervical cancer.

### Antigens.

The peptides used spanning the E7 protein consisted of nine overlapping 22-mer peptides and are indicated by their first and last amino acid in the protein (1-22, 11-32, 21-42, 31-52, 41-62, 51-72, 61-82, 71-92 and 77-98) or 4 long peptides defined by amino acids 1-35, 22-56, 43-77 and 64-98. The peptides spanning the influenza matrix 1 protein of A/PR/8/43 that were used as control peptides in the ELISPOT assay consisted of sixteen 30-mer peptides overlapping by 15 amino acids. Peptides were synthesized and dissolved as described previously (van der Burg *et al.,* 1999).

Recombinant HPV16-E7 protein, HPV16-E6 protein and HIV-1 RT protein (the latter two proteins served as control proteins in proliferation assays) were produced in recombinant *E. coli* transformed with Pet-19b-HPV16-E7, Pet-19b-HPV16-E6 (De Bruijn *et al.,* 1998) or Pet-19b-HIV-1 reverse transcriptase (RT) and purified as described previously (van der Burg *et al.,* 1999).

Memory Response Mix (MRM): A mixture of tetanus toxoid (1 LF/ml; National Institute of Public Health and the Environment, Bilthoven, The Netherlands) and *Mycobacterium tuberculosis* sonicate (2.5µg/ml; generously donated by Dr. P. Klatser, Royal Tropical Institute, The Netherlands ) was used to control the capacity of PBMC to proliferate in response to typical recall antigens.

### HLA-DR-peptide binding assay.

Binding of peptides was measured as reported previously (van der Burg *et al.,* 1999). Briefly, as a source of HLA-DR molecules B-lymphoblastic cell lines homozygous for HLA-DR were used: LG2.1 (DRB*0101, DR1), IWB (DRB1*0201, DR2), HAR (DRB*0301, DR3), BSM (DRB*0401, DR4) and Pitout (DRB1*0701, DR7). DR molecules were purified by affinity chromatography and the purity confirmed by SDS-PAGE. The analysis of peptide binding to purified HLA-DR molecules was performed using N-terminally fluorescencelabeled standard peptides. As standard fluorescent peptides in the binding assays, either HA 308-319 (PKYVKQNTLKLAT, DR1 and DR2), hsp65 3-13 (KTIAYDEEARR, DR3), HA 308-319 Y→F (PKFVKQNTLKLAT, DR4) or Ii 80-103 (LPKPPKPVSKMRMATPLLMQA LPM, DR7) were used.

### Immunogenicity Assay.

HPV16-E7 derived peptide induced proliferation of PBMC isolated from blood obtained from healthy donors was measured as described previously (van der Burg *et al.,* 1999). Briefly, PBMC were seeded at a density of 1.5x10⁵ cells/well of a 96 well U-bottom plate (Costar, Cambridge, MA) in 200 µl of ISCOVE's medium (Gibco) enriched with 10% autologous serum, in the presence or absence of 10µg/ml of indicated 22-mer E7 peptide. As positive control, PBMC were cultured in the presence of a Memory Response Mix. Peptide specific proliferation was measured at day 6 by tritium-thymidine incorporation. Peptides were scored as immunogenic, *i.e.,* able to stimulate T-cells, when the proliferation of >25% of the 8 test wells exceeded the mean proliferation + 2 times the standard deviation of medium control wells.

### Proliferation assays.

Cultures were pulsed with 0.5µCi [³H] thymidine (5Ci/mM, Amersham, UK) per well for 18 hours. Plates were harvested with a Micro cell Harvester (Skatron, Norway). Filters were packed in plastic bags containing 10 ml of scintillation fluid and subsequently counted in a 1205 Betaplate counter (Wallac, Turku, Finland).

MHC class II blocking experiments were carried out as described before using murine monoclonal antibodies against HLA-DQ SPV.L3, against HLA-DR B8.11.2 and against HLA--DP B7/21 (van der Burg *et al.,* 1999). Antibodies were added to APC 1 h prior to protein-APC incubation.

### Isolation and expansion of HPV16-E7-specific T-helper cells.

Peptide-specific T-cell bulk cultures were generated as described previously (van der Burg *et al.,* 1999) using either the E7 22-mer or E7 35-mer peptides. Specific proliferation was measured by incubation of 50,000 responders with an equal amount of irradiated (30Gy) APC (autologous PBMC unless indicated otherwise) and peptide or protein as indicated. E7-peptide and -protein-specific bulk T-cells were cloned by limiting dilution as described previously (van der Burg *et al.,* 1999).

### Cytokine assays.

To determine specific excretion of cytokines, T-cell clones were stimulated by incubation of 50.000 T-cells with an equal amount of APC (30Gy) together with 10µg/ml peptide, control peptide, E7 protein or control protein as indicated. After 24 hours of incubation, supernatant was harvested and replicate wells were pooled. Cytokine production was measured by Enzyme-Linked Immunosorbent Assay (ELISA) as described previously (van der Burg *et al.,* 1999).

### Analysis of antigen-specific T-cells by ELISPOT.

PBMC or cord blood cells (CBC) were seeded at a density of 2 x 10⁶ cells/well of a 24 well plate (Costar, Cambridge, MA) in 1ml of ISCOVE's medium (Gibco) enriched with 10% FCS, in the presence or absence of 5µg/ml of indicated E7-derived 22-mer peptide. As positive control PBMC were cultured in the presence of indicated pools of influenza A/PR/8/34 M1 protein derived peptides consisting of 4 overlapping 30 amino acid long peptides in each pool. Based on our observations, we used a 4-day stimulation before PBMC were transferred to the ELISPOT plates. This resulted in a pronounced IFNγ-production towards influenza M1-derived peptides in the CD45RO+ (memory) subset of T-cells but not in naïve T-cells obtained from adult PBMC (unpublished observations). Following 4 days of incubation at 37°C, PBMC were harvested, washed and seeded in six replicate wells at a density of 10⁵ cells/well of a Multiscreen 96-well plate (Millipore, Etten-Leur, The Netherlands) coated with a IFNγ catching antibody (Mabtech AB, Nacha, Sweden). The ELISPOT was further performed according to the instructions of the manufacturer (Mabtech). The number of spots were analyzed with a fully automated computer assisted video imaging analysis system (Carl Zeiss Vision). Specific spots were calculated by subtracting the mean number of spots + 2xSD of the medium only control from the mean number of spots of experimental wells. Antigen-specific T-cell frequencies were considered to be increased compared to non-responders when T-cell frequencies were ≥ 1/10,000 PBMC.

### Results

### Identification of the immunogenic sequences within HPV16 E7.

We set out to identify the sequences within the HPV16 E7 protein that function as major immunogenic determinants in the context of MHC class II. A set of HPV16 E7 derived overlapping peptides was tested for binding to HLA-DR 1, 2, 3, 4 and 7 in a quantitative peptide/MHC binding assay (Geluk *et al.,* 1995). Together, these HLA-DR molecules cover at least 50-60% of the caucausian, oriental and negroid populations (Baur *et al.,* 1984). Four peptides, E7₁₋₂₂, E7₄₁₋₆₂, E7₅₁₋₇₂ and E7₇₇₋₉₈, bound to three or more different HLA-DR molecules (Table I): peptide E7₁₋₂₂ bound to HLA DR2, 3, 4 and 7, peptide E7₄₁₋₆₂ bound to DR1, 2, 3 and 4 whereas peptide E7₅₁₋₇₂ bound to DR1, 3 and 7 and peptide E7₇₇₋₉₈ bound to DR1, 2 and DR7. This is in accordance with the fact that the peptide binding restrictions for MHC class II molecules were found to be less strict than those for MHC class I (Rammensee *et al.,* 1995). Of note, these peptides failed to bind to one or two other DR molecules tested indicating that their binding, although rather ubiquitous in character, was specific.

Our subsequent experiments focused on the four peptides binding to multiple HLA-DR molecules, as these are the most likely to comprise naturally presented T-helper (Th) epitopes (Geluk *et al.,* 1998). We analyzed whether the peptides could stimulate T-cells to proliferate by adding these to PBMC from 13 HLA-typed healthy blood donors. This assay does not discriminate between the reactivity of memory T-cells and naive, in vitro, primed T-cells but can be readily employed for identification of immunogenic peptides (van der Burg *et al., 1999).* All four peptides were able to stimulate proliferative responses in PBMC from multiple donors (Table I). Most PBMC cultures reacted to the peptides E7₄₁₋₆₂ and E7₅₁₋₇₂ indicating that the central region of the E7 protein harbors the most immunogenic sequences and is likely to be targeted by the immune system in HPV16 infected individuals.

### Mapping of naturally processed T-helper epitopes in HPV16 E7.

As a next step in the identification of Th-epitopes in HPV16 E7, peptide specific bulk T-cell cultures were generated from PBMC of HLA-typed healthy blood donors by repeated stimulation with several long peptides covering the central region of E7. The responding T-cells were subsequently tested for recognition of antigen presenting cells (APC) pulsed with either peptide or whole E7 protein. In the latter setting, presentation of the relevant peptide-epitopes requires antigen-uptake and -processing. Approximately 30% of the Th-cell cultures generated specifically responded to the peptides against which they were raised. Of these, three cultures also showed modest but specific activity against E7 protein pulsed APC, indicating the presence of Th-cells with the capacity to respond against naturally processed antigen (FIGs. 1a, 2a and 3a).

FIG. lb shows the reactivity of a Th-clone that was isolated from HLA-DR15, 4 and DQ6, 7 positive PBMC raised against peptide E7₄₁₋₆₂. These Th-cells specifically respond to APC pulsed with peptide E7₄₁₋₆₂, peptide E7₄₃₋₇₇ or E7 protein. The specificity of the response by this Th-clone exceeded that of the polyclonal culture from which this Th-clone was isolated (compare to FIG. 1a), illustrating that by limiting dilution we have succeeded in isolating the T-cells of interest. Further studies using MHC class II blocking antibodies against HLA-DR, -DQ or -DP (FIG. 1b) and partially MHC class II matched APC revealed that the Th-clone was restricted by HLA-DR15 (FIG. 1c). Fine mapping of the epitope showed that the core sequence recognized was E7₅₀₋₆₂. Furthermore, this Th-clone produced the Th type 1 cytokine IFNγ (FIG. 1d). In a similar fashion a HPV16 E7-specific Th-clone was obtained from HLA-DR3, DQ2 positive PBMC stimulated with peptide E7₂₂₋₅₆ (FIG. 2a). Also this Th-clone secreted IFNγ upon triggering by peptide E7₂₂₋₅₆ and E7 protein pulsed APC (FIG. 2bc). Further analysis showed that this Th-clone recognized the core sequence E7₃₅₋₅₀, as indicated the fact that both peptides E7₃₀₋₅₀ and E7₃₅₋₅₅ recognized, in an HLA-DQ2 restricted fashion (FIG. 2c). A third type 1 cytokine producing Th-clone with distinct specificity was derived from a HLA-DR1, 3 and DQ2 positive PBMC culture stimulated with peptide E7₄₃₋₇₇ (FIG. 3ab). This clone was HLA-DR3 restricted (FIG. 3cd) and produced IFNγ upon recognition of both peptide and protein pulsed APC (FIG. 3e). Whereas peptide E7₄₃₋₇₇ was recognized, the smaller peptide E7₄₁₋₆₂ was not, indicating that the C-terminal part of the E7₄₃₋₇₇ peptide harbors the core epitope. In conclusion, using established Th-clones we mapped three naturally processed epitopes in HPV16 E7. It is conceivable that HPV16+ individuals expressing the relevant MHC class II molecules display in vivo induced Th-immunity against these peptides.

### Memory T-helper responses in subjects with HPV16+ GIN III lesions or cervical carcinoma.

In an approach parallel to the mapping of Th-epitopes using PBMC cultures of healthy individuals, the response against HPV16 E7 in subjects with HPV16+ lesions was analyzed (see Table II for subject characteristics). Primary, in vitro, stimulation of naive T-cells from newborns or adults can result in the production of IL-2 and proliferation of T-cells. However, at this stage such T-cells fail to secrete IFNγ. The production of IFNγ follows when the antigen is encountered again (Early and Reen, 1999; Pittet *et al.,* 1999; Sallusto *et al.,* 1999). This feature allowed us to discriminate between in vitro primed and memory T-cell responses by ELISPOT. Th-responses against the influenza A matrix (M1) protein, which are readily detectable in PBMC of most donors of diverse HLA-types (Table III and our unpublished observations), were measured in parallel as a positive control for the detection of memory T-cell immunity in the assay. Stimulation with M1-peptides of MACS-separated CD45RA+ (naive) T-cells and CD45RO+ (memory) T-cells resulted in the production of IFNγ in the CD45RO+ subset only, confirming that our ELISPOT setup specifically detects memory responses (FIG. 4). Furthermore, cord blood cells (CBC), in accordance with their naive phenotype, did not react by secreting IFNγ when stimulated in vitro with influenza M1 derived peptides. In addition to the PBMC from all healthy donors, those from 9/11 of the HPV16+ subjects reacted to one or more pools of M1-peptides (Table III).

Analysis of HPV16 E7-immunity revealed responses against one or more peptides in 2/3 subjects diagnosed with CIN III and in 3/8 subjects with cervical carcinoma. In addition, one of the seven donors tested displayed immunity against a peptide. The central region of HPV16 E7 that is covered by the peptides E7₃₁₋₅₂, E7₄₁₋₆₂ and E7₅₁₋₇₂ was targeted by the immune system of all five HPV16+ individuals (Table III), not only confirming that this region is highly immunogenic (Table I) but also demonstrating that this region harbors epitopes that are targeted by natural immune responses against HPV16. Interestingly, the specificity of IFNγ responses detected in three HPV16+ subjects, in combination with their HLA-type, matched that of the established Th-clones for which we examined specificity and HLA-restriction in detail (FIGs. 1-3). In particular (Table III, underscored frequencies), subject 2 exhibited significantly increased immunity matching the specificity of the two HLA-DR restricted clones as well as weak immunity matching the HLA-DQ2/E7₃₅₋₅₀ clone. Subject 8 displayed a response matching the DR15/ E7₅₀₋₆₂ clone, whereas subject 11 showed reactivity matching the HLA-DQ2/E7₃₅₋₅₀ clone. It should be noted that in other subjects expressing the relevant MHC class II molecules such responses could not be detected (subjects 3, 4, 7 and 10).

### Discussion

In view of the recently emerged importance of the tumor-specific Th response in the immune defense against solid tumors (reviewed in (Melief *et al.,* 2000; Pardoll and Topalian, 1998; Toes *et al.,* 1999)), and the fact that detailed information on this arm of the immune defense against HPV16 was not yet available, we have conducted studies to analyze HPV16 E7-specific Th-immunity in more detail. Using PBMC cultures from healthy blood donors we have identified the central portion of HPV16 E7 (residues 41-72) as the major immunogenic region with respect to Th-immunity and we have mapped three distinct naturally processed Th-epitopes within this region. In parallel, we have demonstrated the presence of HPV16 E7-specific Th-memory, as detected by IFNγ ELISPOT assays, in several, but not all members of a group of subjects diagnosed with HPV16+ lesions.

We chose to employ IFNγ ELISPOT analysis as a method for measuring natural anti-HPV16-specific immunity in HPV16+ subjects and healthy controls, since the capacity to secrete IFNγ has been reported to discriminate in vivo primed memory T-cells from in vitro primed naïve T-cells (Early and Reen, 1999; Pittet *et al.,* 1999; Sallusto *et al.,* 1999). By exploiting influenza M1-specific memory T-cell responses, commonly present in humans, we have confirmed this issue in that only the CD45RO+ memory T-cell subset was capable of producing IFNγ in our ELISPOT assay. In accordance, we have found E7-specific responses in 5 out of 11 HPV16+ subjects, 4 of which responded against 2 or more peptides, whereas only one of the healthy controls (1/7) displayed reactivity against a single peptide. Since anonymous blood donors were used as controls, and we therefore have no information on the HPV-status and medical history of these subjects, it is possible that also this response reflects natural Th-memory against HPV16. A high number of sexually active women are infected transiently with anogenital HPV types (Ho *et al.,* 1998), which is probably also reflected in the male population (Barrasso *et al.,* 1987).

More importantly, we find that 6 of the HPV16+ subjects do not display HPV16 E7-specific IFNγ responses. In two of these cases (S#6 and 9), this lack of responsiveness may be due to a poor overall condition of the immune response, since these subjects failed to show significant influenza M1-specific immunity (Table III). Such a poor state of the immune system in these subjects can be explained by the fact that these subjects suffered of respectively progressed and recurrent disease (Table II) (Das *et al.,* 1985; Ghosh *et al.,* 1995; Wanebo *et al.,* 1975). Similar observations in cervical cancer patients have previously been reported by several laboratories including our own (Borysiewicz *et al.,* 1996; Ressing *et al.,* 2000). Other HPV16+ subjects, however, fail to show HPV16 E7-directed immunity despite the fact that influenza M1-specific responses are readily detected (Table III; Subjects 3, 4, 7 and 10). Based on the HLA-type of these subjects and our knowledge of the three naturally processed Th-epitopes defined by our HPV16 E7-specific Th-clones, these subjects should be capable of raising T-cell immunity against at least the following Th-epitopes: E7₃₅₋₅₀ (HLA-DQ2; S#7 and 10) and E7₅₀₋₆₂ (HLA-DR15; S#3 and 4). Taken together, these data argue that HPV16 E7-specific T-helper type 1 immunity, although present in several of the HPV16+ subjects, is not markedly induced in other subjects diagnosed with HPV16+ lesions. The absence of these responses can be attributed to various factors. For instance, patients lacking Th1-immunity could instead exhibit Th2-type HPV-specific responses (Clerici *et al.,* 1997). Importantly, our study was performed on a small group of patients, therefore not permitting conclusions concerning the factors underlying absence or presence of HPV-specific Th-immunity. In fact, our findings indicate that Th-immunity in HPV16+ subjects needs to be analyzed further in a more extensive study.

Our current data indicate that IFNγ ELISPOT analysis indeed constitutes a proper methodology for the charting of HPV 16-specific immunity. In parallel, we have performed experiments employing PBMC cultures of healthy blood donors to assist us with the mapping of Th-epitopes in HPV16 E7. These experiments have been rewarding as they have resulted in the identification of the major immunogenic domain in HPV16 E7 as well as in the identification of three naturally processed Th-epitopes and their HLA-restriction elements. Importantly, the specificity of the responses detected by IFNγ ELISPOT analysis in several of the HPV16+ subjects matched up with the specificity and restriction of one or more of our Th-clones, suggesting (but not proving) that the memory responses detected are directed against these defined Th-epitopes. In view of the fact that only part of the HPV16+ subjects display measurable HPV16 E7-specific IFNγ responses, it will be of interest to apply the IFNγ ELISPOT assay for the longitudinal analysis of the anti-HPV16 response in subjects diagnosed with CIN or cervical carcinoma, as well as in subjects that received HPV16-specific vaccination. These studies are currently in progress in our laboratory.

### References

BARRASSO, R., DE BRUX, J., CROISSANT, O. AND ORTH, G., High prevalence of papillomavirus-associated penile intraepithelial neoplasia in sexual partners of women with cervical intraepithelial neoplasia. N. Engl. J. Med., 317, 916-23 (1987).

BAUR, M.P., NEUGEBAUER, M. AND DEPPE, H., Population analysis on the basis of deduced haplotypes from random families. *In:* E.D. Albert, M.P. Baur and W.R. Mayr (eds.), Histocompatibility Testing, pp. 333-341, Springer-Verlag, Berlin (1984).

BORYSIEWICZ, L.K., FIANDER, A., NIMAKO, M., MAN, S., WILKINSON, G.W.G., WESTMORELAND, D., EVANS, A.S., ADAMS, M., STACEY, S.N., BOURSNELL, M.E.G., RUTHERFORD, E., HICKLING, E. AND INGLIS, S.C., A recombinant vaccinia virus encoding human papillomavirus types 16 and 18, E6 and E7 proteins as immunotherapy for cervical cancer. Lancet, 347, 1523-1527 (1996).

BOSCH, F.X., MANOS, M.M., MUNOZ, N., SHERMAN, M., JANSEN, A.M., PETO, J., SCHIFFMAN, M.H., MORENO, V., KURMAN, R. AND SHAH, K.V., Prevalence of human papillomavirus in cervical cancer: a worldwide perspective. International biological study on cervical cancer (IBSCC) Study Group. J Natl Cancer Inst, 87, 796-802 (1995).

CLAAS, B.C., MELCHERS, W.J., VAN DER LINDEN, H.C., LINDEMAN, J. AND QUINT, W.G., Human papillomavirus detection in paraffin-embedded cervical carcinomas and metastases of the carcinomas by the polymerase chain reaction. Am J Pathol, 135, 703-9 (1989).

CLERICI, M., MEROLA, M., FERRARIO, E., TRABATTONI, D., VILLA, M.L., STEFANON, B., VENZON, D.J., SHEARER, G.M., DE PALO, G. AND CLERICI, E., Cytokine production patterns in cervical intraepithelial neoplasia: association with human papillomavirus infection. J.Natl.Cancer Inst., 89, 245-250 (1997).

COLEMAN, N., BIRLEY, H.D., RENTON, A.M., HANNA, N.F., RYAIT, B.K., BYRNE, M., TAYLOR-ROBINSON, D. AND STANLEY, M.A., Immunological events in regressing genital warts. Am. J. Clin. Pathol., 102, 768-74 (1994).

DAS, S.N., KHANNA, N.N. AND KHANNA, S., A multiparametric observation of immune competence in breast cancer and its correlation with tumour load and prognosis. Ann Acad Med Singapore, 14, 374-81 (1985).

DE BRUIJN, M.L., SCHUURHUIS, D.H., VIERBOOM, M.P., VERMEULEN, H., DE COCK, K.A., OOMS, M.E., RESSING, M.E., TOEBES, M., FRANKEN, K.L., DRIJFHOUT, J.W., OTTENHOFF, T.H., OFFRINGA, R. AND MELIEF, C.J., Immunization with human papillomavirus type 16 (HPV16) oncoprotein- loaded dendritic cells as well as protein in adjuvant induces MHC class I-restricted protection to HPV16-induced tumor cells. Cancer Res., 58, 724-731 (1998).

DE GRUIJL, T., BONTKES, H.J., WALBOOMERS, J.M.M., STUKART, M.J., DOEKHIE, F.S., REMMINK, A.J., HELMERHORST, T.J.M., VERHEIJEN, R.H.M., DUGGAN-KEEN, M.F., STERN, P.L., MEIJER, C.J.L.M. AND SCHEPER, R.J., Differential T helper cell responses to human papillomavirus type 16 E7 related to viral clearance or persistence in patients with cervical neoplasia: A longitudinal study. Cancer Res., 58, 1700-1706 (1998).

EARLY, E. AND REEN, D.J., Rapid conversion of naive to effector T cell function counteracts diminished primary human newborn T cell responses. Clin. Exp. Immunol., 116, 527-33 (1999).

EVANS, E.M., MAN, S., EVANS, A.S. AND BORYSIEWICZ, L.K., Infiltration of cervical cancer tissue with human papillomavirus-specific cytotoxic T-lymphocytes. Cancer Res., 57, 2943-2950 (1997).

GELUK, A., TANEJA, V., VAN MEIJGAARDEN, K.E., ZANELLI, E., ABOU-ZEID, C., THOLE, J.E., DE VRIES, R.R., DAVID, C.S. AND OTTENHOFF, T.H., Identification of HLA class II-restricted determinants of Mycobacterium tuberculosis-derived proteins by using HLA-transgenic, class II- deficient mice. Proc.Natl.Acad.Sci.U.S.A., 95, 10797-10802 (1998).

GELUK, A., VAN MEIJGAARDEN, K.E., DRIJFHOUT, J.W. AND OTTENHOFF, T.H., Clip binds to HLA class II using methionine-based, allele-dependent motifs as well as allele-independent supermotifs. Mol.Immunol., 32, 975-981 (1995).

GHOSH, P., KOMSCHLIES, K.L., CIPPITELLI, M., LONGO, D.L., SUBLESKI, J., YE, J., SICA, A., YOUNG, H.A., WILTROUT, R.H. AND OCHOA, A.C., Gradual loss of T-helper 1 populations in spleen of mice during progressive tumor growth. J Natl Cancer Inst, 87, 1478-83 (1995).

HO, G.Y., BIERMAN, R., BEARDSLEY, L., CHANG, C.J. AND BURK, R.D., Natural history of cervicovaginal papillomavirus infection in young women. N. Engl. J. Med., 338, 423-8 (1998).

HOHN, H., PILCH, H., GUNZEL, S., NEUKIRCH, C., HILMES, C., KAUFMANN, A., SELIGER, B. AND MAEURER, M.J., CD4+ tumor-infiltrating lymphocytes in cervical cancer recognize HLA-DR- restricted peptides provided by human papillomavirus-E7. J. Immunol., 163, 5715-22 (1999).

HOPFL, R., WIDSCHWENDTER, A., CHRISTENSEN, N.D., WIELAND, U., ZUMBACH, K., SANDRA, H., PFISTER, H.J., PAWLITA, M. AND HEIM, K., Delayed-type hypersenstivity to HPV16 E7 and humoral immunity to HPV16/18 E6, E7 and HPV 6/11/16/18/31 Li-virus like particles in women with cervical neoplasia. *In:* X. Castellsague, F.X. Bosch, S. de Sanjose, V. Moreno and J. Ribes (eds.), 18th international papillomavirus conference, pp. 299, Institut Catale d'Oncologia, Barcelona, Spain (2000).

MELIEF, C.J.M., TOES, R.E.M., MEDEMA, J.P., VAN DER BURG, S.H., OSSENDORP, F. AND OFFRINGA, R., Strategies for Immunotherapy of Cancer. Adv. Immunol., 75, 235 - 281 (2000).

NAIPAL, A.M., D'AMARO, J., BRUNING, J.W., VAN LEEUWEN, A. AND VAN ROOD, J.J., Automated reading of Propidium Iodide lymphocytotoxicity tests for HLA- DR, MB, MT typing. Tissue Antigens, 24, 302-6 (1984).

NIMAKO, M., FIANDER, A., WILKINSON, G.W.G., BORYSIEWICZ, L.K. AND MAN, S., Human papillomavirus-specific cytotoxic T lymphocytes in patients with cervical intraepithelial neoplasia grade III. Cancer Res., 57, 4855-4861 (1997).

PARDOLL, D.M. AND TOPALIAN, S.L., The role of CD4+ T cell responses in antitumor immunity. Curr. Opin. Immunol., 10, 588-94 (1998).

PITTET, M.J., VALMORI, D., DUNBAR, P.R., SPEISER, D.E., LIENARD, D., LEJEUNE, F., FLEISCHHAUER, K., CERUNDOLO, V., CEROTTINI, J.C. AND ROMERO, P., High frequencies of naive Melan-A/MART-1-specific CD8(+) T cells in a large proportion of human histocompatibility leukocyte antigen (HLA)-A2 individuals. J. Exp. Med., 190, 705-15 (1999).

RAMMENSEE, H.G., FRIEDE, T. AND STEVANOVIIC, S., MHC ligands and peptide motifs: first listing. Immunogenetics, 41, 178-228 (1995).

RESSING, M.E., SETTE, A., BRANDT, R.M.P., RUPPERT, J., WENTWORTH, P.A., HARTMAN, M., OSEROFF, C., GREY, H.M., MELIEF, C.J.M. AND KAST, W.M., Human CTL epitopes encoded by human papillomavirus type 16 E6 and E7 identified through in vivo and in vitro immunogenicity studies of HLA-A*0201 binding peptides. J.Immunol., 154, 5934-5943 (1995).

RESSING, M.E., VAN DRIEL, W.J., BRANDT, R.M., KENTER, G.G., DE JONG, J.H., BAUKNECHT, T., FLEUREN, G.J., HOOGERHOUT, P., OFFRINGA, R., SETTE, A., CELIS, E., GREY, H., TRIMBOS, B.J., KAST, W.M. AND MELIEF, C.J., Detection of T helper responses, but not of human papillomavirus-specific cytotoxic T lymphocyte responses, after peptide vaccination of patients with cervical carcinoma. J. Immunother., 23, 255-66 (2000).

RESSING, M.E., VAN DRIEL, W.J., CELIS, E., SETTE, A., BRANDT, R.M.P., HARTMAN, M., ANHOLTS, J.D.H., SCHREUDER, G.M.T., TER HARMSEL, W.B., FLEUREN, G.J., TRIMBOS, B.J., KAST, W.M. AND MELIEF, C.J.M., Occasional memory cytotoxic T-cell responses of patients with human papillomavirus type 16-positive cervical lesions against a human leukocyte antigen-A*0201-restricted E7-encoded epitope. Cancer Res., 56, 582-588 (1996).

SALLUSTO, F., LENIG, D., FORSTER, R., LIPP, M. AND LANZAVECCHIA, A., Two subsets of memory T lymphocytes with distinct homing potentials and effector functions. Nature, 401, 708-12 (1999).

TOES, R.E.M., OSSENDORP, F., OFFRINGA, R. AND MELIEF, C.J.M., CD4 T cells and their role in antitumor immune responses. J.Exp.Med., 189, 753 - 756 (1999).

VAN DER BURG, S.H., KWAPPENBERG, K.M., GELUK, A., VAN DER KRUK, M., PONTESILLI, O., HOVENKAMP, E., FRANKEN, K.L., VAN MEIJGAARDEN, K.E., DRIJFHOUT, J.W., OTTENHOFF, T.H., MELIEF, C.J. AND OFFRINGA, R., Identification of a conserved universal Th epitope in HIV-1 reverse transcriptase that is processed and presented to HIV-specific CD4+ T cells by at least four unrelated HLA-DR molecules. J. Immunol., 162, 152-60 (1999). WANEBO, H.J., JUN, M.Y., STRONG, E.W. AND OETTGEN, H., T-cell deficiency in patients with squamous cell cancer of the head and neck. Am J Surg, 130, 445-51 (1975).

## Claims

1. An immunogenic epitope selected from the central part of a human papilloma virus 16 E7 protein.

2. An immunogenic epitope according to claim 1, wherein said central part comprises a region spanning amino acid 35-77 of a human papilloma virus 16 E7 protein.

3. An immunogenic epitope according to claim 2, wherein said central part comprises a region spanning amino acid 35-50 in said E7 protein.

4. An immunogenic epitope according to claim 2, wherein said central part comprises a region spanning amino acid 50-62 in said E7 protein.

5. An immunogenic epitope according to claim 2, wherein said central part comprises a region spanning amino acid 43-77 in said E7 protein.

6. A peptide comprising an immunogenic epitope according to claim 1.

7. A peptide according to claim 6, capable of complexing with an MHC class II molecule.

8. A peptide according to claim 7, tested for its capability of eliciting IFNγ production by PBMC derived from an individual diagnosed with CIN III and/or cervical carinoma.

9. A method for generating a human papilloma virus 16 specific T-cell comprising contacting a collection of naive T-cells with a peptide according to anyone of claim 6-8 and culturing at least part of said collection of T-cells.

10. A method for inducing IFNγ production by a human papilloma virus 16 specific memory T-cell comprising contacting a collection of T-cells of a subject infected with human papilloma virus 16 with a peptide according to anyone of claim 6-8 and culturing at least part of said collection of T-cells.

11. A method according to claim 9 or claim 10 further comprising isolating said human papilloma virus 16 specific T-cell.

12. A isolated T cell obtainable by a method according to claim 11.

13. A method for eliciting and/or enhancing an HPV16 E7 protein specific immune response in an individual comprising administering to said individual a peptide according to anyone of claims 5-7 and/or a T-cell according to claim 12.

14. A vaccine comprising a peptide according to any of claims 6-8 and a suitable carrier

15. Use of a T cell according to claim 12, for immunotherapy.

16. Use of a vaccine according to claim 14 or a T cell according to claim 12, for the treatment of a subject comprising an HPV16 lesion.

17. A method for determining whether a collection of T cells comprises a memory T cell specific for an epitope comprising
- providing said collection of T cells with a peptide comprising said epitope,
- culturing said collection of T cells in the presence of an IFNγ antibody and
- detecting any bound IFNγ.
